# EUROPÄISCHE PATENTANMELDUNG

(11) **EP 3 536 327 A1**
(43) Veröffentlichungstag der Anmeldung: **11.09.2019**
(21) Anmeldenummer: 18160911.6
(22) Anmeldetag: 09.03.2018
(51) Int. Cl.: A61K 33/14, A01N 59/00, A61P 33/00, A61P 43/00

(54) **LITHIUM-VERBINDUNGEN ZUR BEKÄMPFUNG VON MILBEN, DIE VON TEILEN DER MENSCHLICHEN ODER WARMBLÜTERISCHEN HAUT LEBEN**

(71) Anmelder: AGILTERA GmbH & Co. KG, 42542 Dormagen (DE)
(72) Erfinder: Heuer, Heike, 41542 Dormagen (DE)
(74) Vertreter: Gille Hrabal

(57) **Zusammenfassung**

Beschrieben wird ein Lithium-haltiges Mittel zur Verwendung bei der prophylaktischen und/oder therapeutischen Behandlung von menschlichen oder warmblüterischen Milbeninfektionen sowie zur Behandlung und/oder Ausrüstung von Oberflächen von Gegenständen.

## Beschreibung

Die vorliegende Erfindung betrifft ein Lithium-haltiges Mittel zur Verwendung bei der prophylaktischen und/oder therapeutischen Behandlung von menschlichen oder warmblüterischen Milbeninfektionen sowie zur Behandlung und/oder Ausrüstung von Oberflächen von Gegenständen. Weiterer Gegenstand der vorliegenden Erfindung ist die Verwendung von Lithium-haltigen Mitteln zur prophylaktischen und/oder therapeutischen Behandlung von menschlichen oder warmblüterischen Milbeninfektionen sowie zur Behandlung und/oder Ausrüstung von Oberflächen von Gegenständen.

Grabmilben (*Sarcoptes spec*.), Haarbalgmilben *(Demodex spec.),* Hausstaubmilben (*Dermatophagoides spec*.) sowie einige eng verwandte Arten leben von Bestandteilen der menschlichen oder warmblüterischen Haut. Ihr Lebensraum ist daher dicht bei Menschen oder Warmblütern. Aufgrund ihrer Winzigkeit und der damit aufgenommenen winzigen Mengen an Haut(bestandteilen) ist ihre Anwesenheit kein materieller Schaden für den besiedelten Organismus oder seine nächste Umgebung. Allerdings scheiden diese Milbenarten Fäkalien aus, in denen sich zum größten Teil unbekannte Stoffe befinden. Je nach Lebensraum führen diese Ausscheidungen nahezu immer (*Sarcoptes-Arten*) oder oft (*Dermatophagoides-Arten*) zu allergischen Reaktionen bei Menschen oder auch Warmblütern, die sich in einem Jucken der Haut oder Augenrötungen, Absonderung von Nasensekret usw. äußern und sich häufig mit Dauer der Einwirkzeit verschlimmern. Man spricht von allergischer Reaktion des Warmblüters auf die genannten Milben (Hausstauballergie, Krätze oder Rosazae). Es gilt daher, ihre Gegenwart zu verhindern.

Da die genannten Milben sehr klein sind, sind sie in der Lage, in kleinste Ritzen und Spalten vorzudringen, die sie sich gegebenenfalls selbst "graben" oder die sie in Matratzen, Bettlaken, sonstigen Textilien oder Materialien der menschlichen Behausung problemlos finden.

Aufgrund der heutigen Lebensweise der Menschen sind diese Milbenarten schwer zu bekämpfen, da häufige Kontakte zwischen Menschen stattfinden, Bewohnungen gleichmäßig beheizt und mit ausreichender Luftfeuchte ausgestattet sind. Zudem müssen die gegen die Milben verwendeten Mittel überall hineindringen, wenn man die Milben chemisch bekämpfen will. Sie werden daher im Allgemeinen physikalisch (Trocknungsprozesse, Kälte- oder Hitzeeinwirkung) teils abgetötet, sind aber nie vollständig zu vernichten. Die Wirkung physikalischer Methoden ist also temporär aber nicht dauerhaft. Das trifft auch für die Vielzahl an bekannten Akariziden (chemische Abtötung) zu, die zwar sehr effizient wirken, aber früher oder später an Wirkung durch chemischen Abbau (Luftoxidation, Verdampfung, etc.) verlieren. Zudem ist es bei den meisten dieser Schädlinge nicht opportun, chemisch/organische Akarizide einzusetzen, da diese auch teils erhebliche Nebenwirkungen auf Menschen entfalten. Gerade allergisierte Patienten meiden daher diese Option.

Aus der WO 2017/042240 A ist bekannt, dass man Lithiummetallsalze zur Behandlung von *Varroa Destructor* in Stöcken von Honigbienen verwenden kann. Andere Anwendungsgebiete sowie andere Milbenformen werden in der Druckschrift nicht erwähnt. Die Erkenntnisse dieser Entgegenhaltung sind auch nicht auf andere Anwendungsgebiete übertragbar, da die Varroa-Milbe den Wirkstoff nicht selbst aufnimmt, sondern "das Blut" der Biene saugt, die den Wirkstoff aufnimmt. Es ist also gar nicht klar, was bei der in der WO 2017/042240 A beschriebenen Anwendung bei Varroa-Milben tatsächlich die Wirkung entfaltet.

Es gilt daher ein Mittel zu finden, welches keinen chemisch-organischen Wirkstoff enthält und zudem dauerhaft gegen Milben wirkt. Die Wirkung sollte den Lebensraum der Milben signifikant einschränken und daher vorzugsweise nachträglich in den Lebensraum der Milben eingebracht werden können oder bevorzugt während des Herstellprozesses von Materialien in diese eingearbeitet werden. Das Mittel sollte sich möglichst nicht durch Luftoxidation verbrauchen oder durch Verdampfung verloren gehen und damit sehr langanhaltend wirksam bleiben.

Darüber hinaus sollte das Mittel in der Lage sein, sowohl auf der menschlichen oder warmblüterischen Haut zu wirken, als auch zur Ausrüstung von Materialien geeignet sein, mit denen Menschen oder Warmblüter in Kontakt kommen können. Hierbei sind insbesondere Anwendungen in Bienenvölkern ausgeschlossen. Dabei sollen die Milben den Wirkstoff selbst aufnehmen und kein intermediärer Organismus, wie in der WO 2017/042240 A beschrieben, zugegen sein.

Erfindungsgemäß wurde nun gefunden, dass bestimmte Lithium-haltige Mittel in der Lage sind, von menschlicher oder warmblüterischer Haut lebende Milben in ihrer Entwicklung stark zu hemmen und letztlich abzutöten. Diese Lithium-haltigen Mittel enthalten als Formulierung einen Lithium-haltigen Bestandteil oder bestehen aus einem Lithium-haltigen Bestandteil, wobei der Lithium-haltigen Bestandteil weiter unten näher beschrieben wird.

Diese Wirkung der Lithium-haltigen Mittel tritt unmittelbar, d.h. bei der Verwendung der reinen Lithium-haltigen Bestandteile, auf. Alternativ können die Lithium-haltigen Bestandteile eine vergleichbare Wirkung entfalten, wenn sie in textile oder polymere Materialien eingearbeitet werden oder mit anderen Stoffen gemischt werden (Formulierungen).

Die vorliegende Erfindung sieht daher die Verwendung von bestimmten Lithium-haltigen Bestandteilen gegenüber Milben vor, wobei der Lithium-haltige Bestandteil sowohl in reiner Form als auch in der Form einer Formulierung gegen Milben eingesetzt werden kann.

Im Nachfolgenden wird vereinfacht von Lithium-haltigen Mitteln gesprochen, wobei diese Lithium-haltigen Mitteln eine Formulierung (Zusammensetzung) sein kann, in welchen der Lithium-haltige Bestandteil, der weiter unten strukturell näher definiert wird, eingearbeitet ist, oder den reinen Lithium-haltigen Bestandteil darstellen können.

Die Wirkung der Lithium-haltigen Mittel, die erst über einige Tage hinweg auftritt, ist dabei ausreichend schnell genug, um die Population an Milben so stark zu dezimieren, dass eine weitere Ausbreitung der Milben im Wesentlichen unterdrückt wird. Dabei inkorporieren die Milben die Lithium-haltigen Mittel oder die durch Lithiumionen verunreinigte Haut/Hautpartikel. Nach dem Ableben der Milben verbleiben die Kadaver der Milben in dem Lebensraum der Milben erhalten, werden mechanisch, chemisch und/oder durch Mikroorganismen zerstört. Die dabei entstehenden Lithiumionen-haltigen Partikel können erneut von Milben aufgenommen werden und führen erneut zum Ableben weiterer Milben. Eine Wirkminderung ist über einen langen Zeitraum nicht zu erwarten.

Die erfindungsgemäßen Lithium-haltigen Mittel sind daher geeignet, menschliche oder warmblüterische Haut sowie Materialien so zu kontaminieren, dass von menschlicher oder warmblüterischer Haut lebende Milben dort nicht mehr existieren können. Da die Anwendungskonzentrationen sehr gering und die partikuläre Bindung der Lithium-haltigen Partikel oder polymeren Strukturen sehr groß ist, gehen von den Lithium-haltigen Partikeln oder dem auf polymeren Strukturen gebundenen Lithiumkomponenten keine gesundheitlichen Gefahren für Menschen aus.

Die vorliegende Erfindung betrifft somit in einem ersten Aspekt ein Lithium-haltiges Mittel zur Verwendung bei der prophylaktischen und/oder therapeutischen Behandlung von menschlichen oder warmblüterischen Milbeninfektionen sowie zur Behandlung und/oder Ausrüstung von Oberflächen von Gegenständen.

Das erfindungsgemäße Lithium-haltige Mittel enthält einen Lithium-haltigen Bestandteil, der ausgewählt ist aus der Gruppe, bestehend aus
- Lithiumhydroxid;
- Umsetzungsprodukte, die durch das Verseifen von natürlichen Fetten und Ölen mit Lithiumhydroxid (sogenannte Lithiumseifen) entstehen;
- Lithiumsalze mineralischer Säuren;
- Lithiumsalze von Carboxymethylcellulose;
- Lithium-Salze von Polyethylenglykolcarbonsäuren;
- Lithiumsalze organischer Säuren der allgemeinen Formel
   Li-CO₂-(CH₂)ₙ-H mit n = 0 bis 30,
   wobei der Alkylrest der Carbonsäuren geradlinig oder verzweigt, gesättigt oder ungesättigt sowie unsubstituiert oder substituiert sein kann;
- Lithiumchlorid, Lithiumbromid, Lithiumsulfat, Lithiumnitrat, Lithiumcarbonat und Lithiumphosphat; und
- Mischungen der vorstehenden Lithium-haltigen Bestandteile.

In einer bevorzugten Ausführungsform wird der Lithium-haltige Bestandteil ausgewählt aus der Gruppe, bestehend aus
- Lithiumhydroxid;
- Umsetzungsprodukte, die durch das Verseifen von natürlichen Fetten und Ölen mit Lithiumhydroxid (sogenannte Lithiumseifen) entstehen;
- Lithiumsalze von Carboxymethylcellulose;
- Lithium-Salze von Polyethylenglykolcarbonsäuren; und
- Mischungen der vorstehenden Lithium-haltigen Bestandteile.

In einer weiteren bevorzugten Ausführungsform ist der Lithium-haltige Bestandteil Lithiumhydroxid.

### Definitionen

Der Begriff" Prophylaxe" umfasst Maßnahmen zur Verhütung und Vorbeugung von Krankheiten und zur Vermeidung von Krankheitsfolgen, insbesondere von Milbenbefall. Die Begriffe "Prävention", "Prophylaxe" oder "Vorbeugung" werden im Rahmen der vorliegenden Erfindung synonym verwendet und bezeichnen das Vermeiden oder Vermindern des Risikos, einen Milbenbefall zu bekommen, zu erfahren, zu erleiden oder zu haben.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Milbeninfektion.

Die Behandlung oder die Prävention eines Milbenbefalls können im Rahmen der vorliegenden Erfindung teilweise oder vollständig erfolgen.

Der Begriff "Lithium-haltiger Bestandteil" bezeichnet vorliegend denjenigen Stoff in einem Lithium-haltigen Mittel, dem dessen milbenbekämpfende Wirkung zugeschrieben wird. Meist wird der Lithium-haltige Bestandteil in Kombination mit einem oder mehreren Hilfsstoffen, gelegentlich aber auch ohne Hilfsstoffe, zum Lithium-haltigen Mittel verarbeitet.

Unter dem Begriff der Warmblüter (gleichwarmes Tier oder homoiothermes Tier) werden im Rahmen der vorliegenden Erfindung in Analogie zur Zoologie Tiere (d.h. Vögel und Säugetiere) bezeichnet, die ihre Körperkerntemperatur unabhängig von der Umwelttemperatur auf einen konstanten Temperaturwert regulieren können.

Als ein möglicher Lithium-haltiger Bestandteil werden Lithiumseifen genannt. Im Rahmen der vorliegenden Erfindung werden unter Lithiumseifen Lithiumsalze einzelner Fettsäuren oder Gemische von Lithiumsalzen mehrerer Fettsäuren verstanden. Die Verseifung natürlicher Fette und Öle mit Lithiumhydroxid liefert Gemische von Lithiumsalzen der Fettsäuren und Glycerin. Die Anteile der einzelnen Fettsäure-Anionen im Gemisch der Lithiumsalze hängen dabei von der Natur und Provenienz des als Rohstoff verwendeten Triglycerid ab. Eine chemisch weitgehend einheitliche Lithiumseife kann man erhalten durch Umsetzung einer reinen Fettsäure mit einer stöchiometrischen Menge Lithiumhydroxid.

Die vorliegende Erfindung richtet sich insbesondere zur prophylaktischen und/oder therapeutischen Behandlung von menschlichen oder warmblüterischen Milbeninfektionen sowie zur Behandlung und/oder Ausrüstung von Oberflächen von Gegenständen zum Schutz vor Grabmilben (*Sarcoptes spec*.), Haarbalgmilben (*Demodex spec*.) und Hausstaubmilben (*Dermatophagoides spec*.).

### Nähere Beschreibung der Erfindung

Die Auswahl des bevorzugten Lithium-haltigen Bestandteils hängt erfindungsgemäß von dem gewünschten Einsatzzweck ab und wird weiter unten näher erläutert.

In einer ersten Ausführungsform wird das erfindungsgemäße Mittel zur oberflächlichen Behandlung der menschlichen und warmblüterischen Haut verwendet.

In einer zweiten Ausführungsform wird das erfindungsgemäße Mittel zur Behandlung und/oder Ausrüstung von Oberflächen von Gegenständen, die insbesondere mit Menschen oder Warmblütern in Kontakt stehen, verwendet.

Im Nachfolgenden werden diese beiden Ausführungsformen näher beschrieben.

### Erste Ausführungsform

Die vorliegende Erfindung betrifft in der ersten Ausführungsform ein Lithium-haltiges Mittel zur Verwendung bei der prophylaktischen und/oder therapeutischen Behandlung von menschlichen oder warmblüterischen Milbeninfektionen.

Bestimmte Arten der Milben leben auf und von der Haut, die abgestorbene Teile enthält, aber noch mit dem lebenden Körper von Warmblütern oder Menschen verbunden ist. Diese Arten, beispielsweise Grabmilben (Sarcoptes spec.) und Haarbalgmilben (Demodex spec.), lassen sich durch das erfindungsgemäße Lithium-haltige Mittel effizient bekämpfen. Die Anwendung insbesondere von der Base Lithiumhydroxid auf der Haut führt aber gerade, wenn die Anwendung längere Zeit durchgeführt werden muss, zu Hautschäden. Sie lassen sich vermeiden, wenn Lithiumseifen verwendet werden, also Lithiumsalze längerer Fettsäuren, Lithiumsalze von Carboxymethylcellulose, Lithium-Salze von Polyethylenglykolcarbonsäuren etc. Die einfachste Art und Weise, die abgestorbene Haut mit Lithium zu im Sinne der vorliegenden Erfindung zu kontaminieren ist, dabei das Verwenden eines kommerziellen Hautpflegemittels oder bekannter Rezeptur zur Behandlung von Haut und Zusatz des erfindungsgemäßen Lithium-haltigen Mittels in einer gewünschten Menge. Beispiele konkreter Salben werden untenstehend beschrieben.

Salze des Lithiums, die ausreichend wasserlöslich sind und sich in die Hautpflegemittel einarbeiten lassen, sind ebenso geeignet.

Hierbei handelt es sich zunächst einmal bevorzugt um Verbindungen der allgemeinen Formel
Li-CO₂-(CH₂)ₙ-H mit n = 0 bis 30,
wobei der Alkylrest der Carbonsäuren geradlinig oder verzweigt, gesättigt oder ungesättigt sowie unsubstituiert oder substituiert sein kann. Geeignete Carbonsäuren für diese Zwecke sind Aminosäuren, Ameisensäure, Essigsäure, Buttersäure, Stearinsäure, Zitronensäure und Milchsäure.

Beispiele für konkrete Lithiumsalze in diesem Zusammenhand sind Lithiumformiat, Lithiumacetat, Lithiumpropionat, Lithiumbutyrat und Lithiumstearat.

Weitere Beispiele für Salze, die in Hauptpflegemittel eingearbeitet werden können, werden ausgewählt aus der Gruppe, bestehend aus Lithiumchlorid, Lithiumbromid, Lithiumsulfat, Lithiumnitrat, Lithiumcarbonat und Lithiumphosphat.

Die vorstehend genannten Lithium-haltigen Mittel werden in einer für die warmblüterische oder menschliche Haut verträglichen Formulierung bevorzugt appliziert. Die Lithiumionen dringen bei der Behandlung dabei in die oberen, abgestorbenen Hautschichten ein und werden von den Grabmilben (Sarcoptes spec.) oder Haarbalgmilben (Demodex spec.) mit aufgenommen, was ihren Lebenzeitraum verkürzt und bei ausreichender Dosierung innerhalb von Tagen oder wenigen Wochen zum Absterben bringt.

Die Konzentration an Lithiumionen beträgt vorzugsweise 1000 ppm bis 0,1 ppm, weiter bevorzugt 500 ppm bis 1 ppm noch weiter bevorzugt 250 ppm bis 5 ppm.

Zur Konzentration der wirksamen Lithiumdosierung ist anzumerken, dass diese sehr gering sein kann. Eine Dosierung von 1 mg/kg Lithiumionen, bezogen auf die verwendete Formulierung ist bereits wirksam, wenn die Einwirkzeit ausreichend lange (ca. 14 Tage) ist. Soll die Wirkung schneller eintreten, ist die Dosierung zu erhöhen, wobei eine Konzentration von bis zu 300 mg/kg Lithiumionen sinnvoll sein kann. Die Konzentrationen sind jeweils auf das von den Milben aufzunehmende Substrat bemessen.

Wenn die erfindungsgemäßen Lithium-haltigen Bestandteile in einer topisch applizierbaren Formulierung verwendet werden, so kann die Formulierung neben dem Lithium-haltigen Bestandteil zusätzlich Methylsalicylat, Kampfer, Menthol, mikrokristallines Paraffin, Wollwachs, Hydroxyethlcellulosegel, Glycerinmonsterat 60, Cetylalkohol, mittelkettige Triglyceride, Vaselin, Macrogol-20-glycerolmonostearat, Propylenglykol, Wasser, Harnstoff, Vaseline, Zinkoxid, Weizenstärke, Ölsäure und Wasser enthalten.

Die Zubereitung kann in der Form eines Hautpflegemittels, insbesondere in der Form einer Salbe, einer sprühbaren Zubereitung, einer Lösung, einer Lotion oder einer Creme, vorliegen, wenn eine topische Anwendung am Menschen oder Warmblüter vorgesehen ist.

Eine Applizierung mit Lithiumionen kann auch dergestalt erfolgen, dass das Lithium-haltige Mittel als (Tauch-)bad bis zu einem pH-Wert von ungefähr 9 verwendet wird.

### Zweite Ausführungsform

In einer zweiten Ausführungsform der vorliegenden Erfindung wird das Lithium-haltige Mittel zur Behandlung und/oder Ausrüstung von Materialien verwendet. Bei diesen Materialien handelt es sich insbesondere um Gegenstände, die in Kontakt mit Menschen und/oder Warmblütern stehen. Ausgeschlossen sind hierbei Bienenstöcke.

Zu schützenden Materialien, die im Rahmen der vorliegenden Erfindung mit den vorstehenden Lithium-haltigen Mitteln entweder behandelt oder ausgestattet werden können, sind beispielsweise Materialien auf Polymerbasis, wie auf Basis von Polyurethan, Polyester, Polyamid, Zellulose und Baumwolle. Diese Materialien bilden mit den erfindungsgemäßen Lithium-haltigen Mitteln Gemische und/oder Lithium-haltige Oberflächen, so dass der Lebensraum der Milben mit Lithium durchdrungen wird. Konkrete Beispiele für zu behandelnde Materialien sind beispielsweise Matratzen und Bettzeug, die beispielsweise aus natürlichem oder synthetischem Latex, Kokosfasern, Rosshaar, Stroh, Seegras, Baumwolle, Schur- oder Schafswolle, Seide, Federn, Vlies sowie aus synthetischen Schaumstoffen auf Basis von Polyurethanen, Viskose usw. bestehen.

Die Materialeigenschaften und die Verarbeitung dieser für Matratzen und Bettzeug verwendeten Materialien sind so gewählt, dass die Matratze eine hohe Flexibilität aufweist. Dabei ist Luftzufuhr und Feuchtigkeit in und auf der Matratze ideal geeignet Milben zu beheimaten. Die regelmäßige Zufuhr von Hautschuppen stellt dann die Ernährung und Vermehrung der Milben sicher.

Aber auch im Oberbett, also im Bettlaken, im Federbett, Kopfkissen oder im Bett getragener "Bettkleidung" wie Bettschuhe finden sich viele Milben und können dort, ebenso wie in Polstermöbeln und Teppichen aber auch in Büchern, mit den erfindungsgemäßen Lithium-haltigen Mitteln bekämpft werden.

Das erfindungsgemäße Lithium-haltige Mittel ist insbesondere für die Behandlung von Matratzen geeignet, weil sich dort regelmäßig sehr hohe Ansammlungen von Hausstaubmilben finden.

Letztlich sind zwei chemische Strukturelemente der vorstehenden Materialien erkennbar, mit denen das Lithium-haltige Mittel wechselwirkt und gebunden wird:
- R₁-CO-NH-R₂ wie in Naturlatex (Eiweißanteil), Ross- und anderen Haaren, (Vogel)-Federn, Kokosfasern (Eiweißanteil), Stroh (Eiweißanteil), Seegras (Eiweißanteil), Schurwolle, Schafswolle, Vlies, Seide, Polyurethanen, Polyamide, Polyacrylamid, Polyacrylnitril (Amid-Endgruppen) etc.: Die Stabilität von Lithium-haltigen Mitteln in Materialien, welche das vorgenannte Strukturelement enthalten, kann durch vermutete Ab- und Adsorption an die Amidgruppe begründet werden. Dieser Bindungstyp wird durch die Verwendung von Fleisch- und Hefeextrakt in den unter stehenden Beispielen wiedergegeben.
- Polyalkohole, wie in den Zelluloseanteilen von Kokosfasern, Stroh, Seegras, Viskose: Die Stabilität von Lithium-haltigen Mitteln in Materialien, welche das vorgenannte Strukturelement enthalten, kann durch natürliche Oxidationsprozesse begründet werden, die in den polymeren Strukturen Ketone und höher oxidierte Einheiten schafft, die das Lithium binden, ad- oder absorbieren. Hierfür ist Lithiumhydroxid bestens geeignet, andere Lithiumverbindungen aber je nach Löslichkeit oder Struktur ebenso. Dieser Bindungstyp wird durch die Verwendung von Baumwolle sowie von Malzextrakt in den unter stehenden Beispielen wiedergegeben.

Materialien, die keines er genannten Strukturelemente enthalten, wie zum Beispiel synthetischer Latex, Polypropylen, Polyethylen, Polyester, PVC, Polystyrol, PTFE etc. sind zu dieser verstärkenden Anlagerung von Lithiumhydroxid nicht in der Lage. Die Wirkung des Lithium-haltigen Mittels ist dadurch prinzipiell nicht eingeschränkt, hält aber nicht so lange an, da das Lithium-haltige Mittel schneller als in den o.g. Strukturen verloren geht. Um die Haftfähigkeit zu verbessern, ist zum Beispiel der Zusatz von Carboxymethylcellulose (CMC) möglich und sinnvoll. Ebenfalls kann eine solche Oberfläche mit langkettigen Fettsäuresalzen des Lithiums imprägniert werden, um den Lebensraum der Milben zu zerstören.

Im Fall der Verwendung des erfindungsgemäßen Lithium-haltigen Mittels zur Ausrüstung von Materialien ist das erfindungsgemäße Lithium-haltige Mittel entweder während des Herstellprozesses in das Material einzuarbeiten oder später aufzutragen.

Ein Einarbeiten des Lithium-haltigen Mittels kann zum Beispiel durch Zugabe der vorstehenden Lithium-haltigen Mittel in den Polymerisationsprozess oder beim Behandeln der Materialien im finalen Herstellprozess, z. B. bei einer Waschung, einer Reinigung oder einem Finishing, erfolgen, so dass ausreichende Mengen an Lithiumionen im Material verbleiben.

Auch ein Einsprühen der Oberflächen der zu behandelnden Materialien mit einer Lösung, welche den erfindungsgemäßen Lithium-haltigen Bestandteil enthält, ist möglich.

Die Behandlung bzw. Ausrüstung der vorstehenden Materialien mit Lithiumionen erfolgt vorzugsweise dergestalt, dass die Konzentration an Lithiumionen in dem Material bzw. auf dem Material vorzugsweise 0,1 bis 1000 ppm, weiter bevorzugt 1 bis 500 ppm, noch weiter bevorzugt 10 bis 250 ppm beträgt.

Die Behandlung bzw. Ausrüstung der vorstehenden Materialien mit Lithiumionen kann auch dergestalt erfolgen, dass das Lithium-haltige Mittel als (Tauch-)bad bis zu einem pH-Wert von ungefähr 9 aufgetragen wird.

Ein weiterer Gegenstand der vorliegenden Erfindung ist die Verwendung von mindestens einen Lithium-haltigen Bestandteil, ausgewählt aus der Gruppe, bestehend aus
- Lithiumhydroxid;
- Umsetzungsprodukte, die durch das Verseifen von natürlichen Fetten und Ölen mit Lithiumhydroxid entstehen;
- Lithiumsalze mineralischer Säuren;
- Lithiumsalze von Carboxymethylcellulose;
- Lithium-Salze von Polyethylenglykolcarbonsäuren;
- Lithiumsalze organischer Säuren der allgemeinen Formel
   Li-CO₂-(CH₂)ₙ-H mit n = 0 bis 30,
   wobei der Alkylrest der Carbonsäuren geradlinig oder verzweigt, gesättigt oder ungesättigt sowie unsubstituiert oder substituiert sein kann;
- Lithiumchlorid, Lithiumbromid, Lithiumsulfat, Lithiumnitrat, Lithiumcarbonat und Lithiumphosphat; und
- Mischungen der vorstehenden Lithium-haltigen Bestandteile.
bei der prophylaktischen und/oder therapeutischen Behandlung von menschlichen oder warmblüterischen Milbeninfektionen sowie zur Behandlung und/oder Ausrüstung von Oberflächen von Gegenständen.

Bezüglich dieser erfindungsgemäßen Verwendung wird zwecks Vermeidung von Wiederholungen auf oben stehende Ausführungen verwiesen.

Die vorliegende Erfindung wird anhand der nachfolgenden Beispiele näher erläutert.

### Beispiele:

Es werden Lösungen von Lithiumhydroxid und verschiedenen Komponenten hergestellt.

| **Zubereitung** | **Kennzeichnung** | **Zusammensetzung** | **EndpH-Wert** |
|---|---|---|---|
| 1 | 1CL | 2,7744 g Zitronensäure in 50 ml destilliertem Wasser und Zusatz von Lithiumhydroxid | 8,5 |
| 2 | 2ML | 50 ml Mediumlösung 1 und Zusatz von Lithiumhydroxid | 8,5 |
| 3 | 3CML | 50 ml Mediumlösung 1 und 2,9873 g Zitronensäure und Zusatz von Lithiumhydroxid | 8,5 |

Die Mediumlösung besteht aus 2,9873 g Hefeextrakt, 2,5221 g Malzextrakt und 2,6449 g Fleischextrakt in 100 ml destilliertem Wasser. Die Zubereitungen sind partikelfrei über einen 0,2 µ Filter filtriert.

Es wurden die Lösungen genannter Zusammensetzung hergestellt und mit so viel Lithiumhydroxid versetzt, bis der Ziel-pH-Wert erreicht war.

Als Testorganismus wird Dermatophagoides pteronyssinus (Hausstaubmilbe) eingesetzt. Die Testbedingungen erfolgen unter den Vorgaben der Biocidal Products Regulation Volume II Efficiency-Assessment and Evaluation (Parts B&C), Version 1.0, February 2017, ECHA.

Dazu wird die Zubereitung auf einen Baumwollstoff durch feinverteilendes Aufsprühen appliziert, so dass dieser vollständig oberflächig benetzt wird. Dazu werden pro Replikat 6 Sprühstöße der entsprechenden Zubereitung auf den Baumwollstoff aufgesprüht. Der Baumwollstoff wird nach Trocknung alsdann in einer Petrischale eingebracht, wobei der obere Rand der Schale durch eine Teflonschicht gegen den Ausbruch von Milben gesichert ist. Adulte Milben (weibliche und männliche Tiere, in Summe 20 Tiere pro Petrischale) werden dann mit einer Nadel in dem so konzipierten "Lebensraum" eingetragen und bei 25 °C und 75 % relativer Luftfeuchte (Idealbedingungen für die Milben) für 24 h, 3 Tagen und 6 Tagen beobachtet und abgemustert. Das Überleben der Milben auf dem Baumwollstoff wird anhand der Mortalität bewertet. Eine Milbe gilt als verstorben, so denn keinerlei Bewegung mehr beobachtet wird.

Jeder Test wird in dreifacher Replikation durchgeführt. Als Blindprobe werden Milben unter identischen Bedingungen gehalten, wobei als Sprühlösung reines destilliertes Wasser verwendet wird. Der Test gilt als gültig, wenn im Blindversuch weniger als 20 % der Milben versterben.

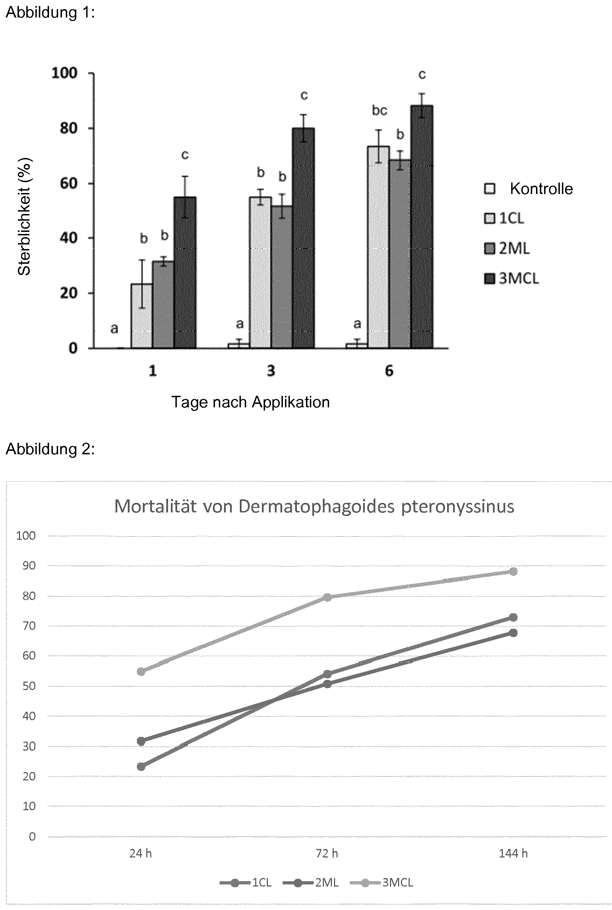

In der Abbildung 1 wird gezeigt: Sterblichkeit (Mittelwert ± SE) von Dermatophagoides pteronyssinus Milben nach 1, 3 und 6 Tagen Exposition gegenüber den Testprodukten 1CL, 2ML, 3MCL und entionisiertem Wasser (Kontrolle) auf Baumwollgewebe gesprüht. Unterschiedliche Buchstaben zeigen signifikante Unterschiede (P <0,05) in der Anzahl der überlebenden Milben zwischen den Testgruppen innerhalb des jeweiligen Beobachtungstages nach Fisher's genauen Tests mit Bonferroni-Korrektur für Mehrfachvergleiche. N _{Behandlung} = 3; N _{Milben} = 60.

**Tabelle 2: Relative Wirksamkeit (Wirksamkeit korrigiert für die Kontrolle) der Testprodukte 1CL, 2ML und 3MCL gegen Dermatophagoides pteronyssinus Milben nach 1, 2 und 3 Tagen Exposition auf Baumwollgewebe. Signifikanzen sind nach Bonferroni korrigierten Fisher's genauen Tests zur Anzahl der überlebenden Milben in Testprodukt- und Kontrollläufen. ***: P <0,001. N _{Behandlung} = 3; N _{Milben} = 60.**

| Test Produkt | Relative Wirksamkeit (%) 1T | Signifikanz 1T | Relative Wirksamkeit (%) 3T | Signifikanz 3T | Relative Wirksamkeit (%) 6T | Signifikanz 6T |
|---|---|---|---|---|---|---|
| **1CL** | 23.3 | *** | 54.2 | *** | 72.9 | *** |
| **2ML** | 31.7 | *** | 50.8 | *** | 67.8 | *** |
| **3MCL** | 55.0 | *** | 79.7 | *** | 88.1 | *** |

Alle Tests konnten signifikant abgeschlossen werden. Es ist erkennbar, dass das Einwirken der Zubereitungen nach einem Tag erwartungsgemäß keine ausreichende Wirkung entfaltet, hingegen aber nach schon sechs Tagen eine Mortalität dicht bei 90 % erreicht wird.

Durch Extrapolation ist erkennbar, dass nach spätestens einer Woche eine Mortalität von 90 % überschritten wird. Für eine vorbeugende Behandlung von Komponenten, die ein Bett oder eine andere üblicherweise von Milben bewohntes Gebiet sicher von Milbenbefall befreien soll, ist eine Wirkung von >90 % in vier Wochen ausreichend, da das dem Generationswechsel entspricht. Wird eine Generation erfasst, ist nicht mit neuen Milben zu rechnen. Wie oben ausgeführt, werden die vorgeschlagenen Zubereitungen aber auch darüber hinaus wirksam bleiben, da sich der Wirkstoff nicht verbraucht.

Herstellung einer Zubereitung zur Anwendung auf lebenden Menschen/Warmblütern:
Salbe 1 :
   10 Teile Methylsalicylat, 10 Teile racemischer Kampfer, 2 Teile Menthol, 20 Teile mikrokristallines Paraffin 90°C und zu 100 Teilen Wollwachssalbe DAB werden gemäß NRF 1.2 angerührt und mit Lithiumhydroxid auf pH > 8 eingestellt. Die Salbe wird auf dem ganzen Körper des mit Grabmilben (Sarcoptes spec.) oder im Kopfbereich des mit Haarbalgmilben (Demodex spec) befallenden Menschen oder dem ganzen Körper des Warmblüters gleichmäßig eingerieben. Die Einreibung wird täglich wiederholt. Der Befall mit Milben ist nach 6 Tagen nicht mehr nachweisbar.
Salbe 2:
   Hydroxyethlcellulosegel DAB wird mit 0,8 % Lithiumhydroxid versetzt. Die Salbe wird auf dem ganzen Körper des mit Grabmilben (Sarcoptes spec.) oder im Kopfbereich des mit Haarbalgmilben (Demodex spec) befallenden Menschen oder dem ganzen Körper des Warmblüters gleichmäßig eingerieben. Die Einreibung wird täglich wiederholt. Der Befall mit Milben ist nach 8 Tagen nicht mehr nachweisbar.
Salbe 3:
   Basiscreme DAC (aus Glycerinmonsterat 60, Cetylalkohol, mittelkettige Triglyceride, weißes Vaselin, Macrogol-20-glycerolmonostearat, Propylenglykol, gereinigtem Wasser) wird mit 0,4 % Lithiumhydroxid versetzt. Die Salbe wird auf dem ganzen Körper des mit Grabmilben (Sarcoptes spec.) oder im Kopfbereich des mit Haarbalgmilben (Demodex spec) befallenden Menschen oder dem ganzen Körper des Warmblüters gleichmäßig eingerieben. Die Einreibung wird täglich wiederholt. Der Befall mit Milben ist nach 12 Tagen nicht mehr nachweisbar.
Salbe 4:
   20 Teile Harnstoff, 40 Teile Vaseline und auf 100 Teile ergänzt Wollwachs wird mit 0,3 % Lithiumhydroxid versetzt. Die Salbe wird auf der befallenden Körperstelle des mit Grabmilben (Sarcoptes spec.) befallenden Menschen einmalig gleichmäßig eingerieben. Die weitere Behandlung erfolgt mit den Salben 1 - 3. Der Befall mit Milben ist nach 8 Tagen nicht mehr nachweisbar.
Salbe 5:
   20 Teile Zinkoxid, 10 Teile Weizenstärke, 10 Teile mittelkettige Triglyceride, 0,3 Teil Ölsäure, 30 Teile Wollwachs und zu 100 Teilen ergänzt weißes Vaseline (gemäß NRF 11.3 hergestellt) werden mit Lithiumhydroxid versetzt, bis 7 > pH > 8. Die Salbe wird auf dem ganzen Körper des mit Grabmilben (Sarcoptes spec.) oder im Kopfbereich des mit Haarbalgmilben (Demodex spec) befallenden Menschen oder dem ganzen Körper des Warmblüters gleichmäßig eingerieben. Die Einreibung wird täglich wiederholt. Der Befall mit Milben ist nach 10 Tagen nicht mehr nachweisbar.

## Patentansprüche

1. Lithium-haltiges Mittel zur Verwendung bei der prophylaktischen und/oder therapeutischen Behandlung von menschlichen oder warmblüterischen Milbeninfektionen sowie zur Behandlung und/oder Ausrüstung von Oberflächen von Gegenständen, umfassend mindestens einen Lithium-haltigen Bestandteil, ausgewählt aus der Gruppe, bestehend aus Lithiumhydroxid; Umsetzungsprodukte, die durch das Verseifen von natürlichen Fetten und Ölen mit Lithiumhydroxid entstehen; Lithiumsalze mineralischer Säuren; Lithiumsalze von Carboxymethylcellulose; Lithium-Salze von Polyethylenglykolcarbonsäuren; Lithiumsalze organischer Säuren der allgemeinen Formel
Li-CO₂-(CH₂)ₙ-H mit n = 0 bis 30,
wobei der Alkylrest der Carbonsäuren geradlinig oder verzweigt, gesättigt oder ungesättigt sowie unsubstituiert oder substituiert sein kann; Lithiumchlorid, Lithiumbromid, Lithiumsulfat, Lithiumnitrat, Lithiumcarbonat und Lithiumphosphat; und Mischungen der vorstehenden Lithium-haltigen Bestandteile.

2. Lithium-haltiges Mittel nach Anspruch 1, **dadurch gekennzeichnet, dass** das Lithium-haltige Bestandteil ausgewählt ist aus der Gruppe, bestehend aus Lithiumhydroxid; Umsetzungsprodukte, die durch das Verseifen von natürlichen Fetten und Ölen mit Lithiumhydroxid entstehen; Lithiumsalze von Carboxymethylcellulose; Lithium-Salze von Polyethylenglykolcarbonsäuren; und Mischungen der vorstehenden Lithium-haltigen Bestandteile.

3. Lithium-haltiges Mittel nach Anspruch 1 oder 2, **dadurch gekennzeichnet, dass** das Lithium-haltige Mittel ein Hautpflegemittel, insbesondere in der Form einer Salbe, einer sprühbaren Zubereitung, einer Lösung, einer Lotion oder einer Creme, ist.

4. Lithium-haltiges Mittel nach einem der Ansprüche 1 bis 3, **dadurch gekennzeichnet, dass** der Lithium-haltige Bestandteil wasserlöslich ist.

5. Lithium-haltiges Mittel nach Anspruch 1 oder 2, **dadurch gekennzeichnet, dass** die Gegenstände Materialien auf Polymerbasis, insbesondere auf Basis von Polyurethan, Polyester, Polyamid, Zellulose und Baumwolle, umfassen.

6. Lithium-haltiges Mittel nach Anspruch 5, **dadurch gekennzeichnet, dass** die zu behandelnden und/oder auszurüstenden Gegenstände ausgewählt sind aus der Gruppe, bestehend aus Matratzen und Bettzeug.

7. Lithium-haltiges Mittel nach Anspruch 6, **dadurch gekennzeichnet, dass** die Matratzen und das Bettzeug aus natürlichem oder synthetischem Latex, Kokosfasern, Rosshaar, Stroh, Seegras, Baumwolle, Schur- oder Schafswolle, Seide, Federn, Viskose, Vlies und/oder aus synthetischen Schaumstoffen auf Basis von Polyurethanen bestehen.

8. Lithium-haltiges Mittel nach einem der Ansprüche 1 bis 7, **dadurch gekennzeichnet, dass** das Lithium-haltige Mittel zum Schutz vor Grabmilben (*Sarcoptes spec*.), Haarbalgmilben (*Demodex spec*.) und Hausstaubmilben (*Dermatophagoides spec*.) dient.

9. Verwendung von einem Lithium-haltigen Mittel bei der prophylaktischen und/oder therapeutischen Behandlung von menschlichen oder warmblüterischen Milbeninfektionen sowie zur Behandlung und/oder Ausrüstung von Oberflächen von Gegenständen, umfassend mindestens einen Lithium-haltigen Bestandteil, ausgewählt aus der Gruppe, bestehend aus Lithiumhydroxid; Umsetzungsprodukte, die durch das Verseifen von natürlichen Fetten und Ölen mit Lithiumhydroxid entstehen; Lithiumsalze mineralischer Säuren; Lithiumsalze von Carboxymethylcellulose; Lithium-Salze von Polyethylenglykolcarbonsäuren; Lithiumsalze organischer Säuren der allgemeinen Formel
Li-CO₂-(CH₂)ₙ-H mit n = 0 bis 30,
wobei der Alkylrest der Carbonsäuren geradlinig oder verzweigt, gesättigt oder ungesättigt sowie unsubstituiert oder substituiert sein kann; Lithiumchlorid, Lithiumbromid, Lithiumsulfat, Lithiumnitrat, Lithiumcarbonat und Lithiumphosphat; und Mischungen der vorstehenden Lithium-haltigen Bestandteile.

10. Verwendung nach Anspruch 9, **dadurch gekennzeichnet, dass** das Lithium-haltige Mittel ein Hautpflegemittel, insbesondere in der Form einer Salbe, einer sprühbaren Zubereitung, einer Lösung, einer Lotion oder einer Creme, ist.

11. Verwendung nach Anspruch 9 oder 10, **dadurch gekennzeichnet, dass** die Gegenstände Materialien auf Polymerbasis, insbesondere auf Basis von Polyurethan, Polyester, Polyamid, Zellulose und Baumwolle, umfassen und/oder die zu behandelnden und/oder auszurüstenden Gegenstände ausgewählt sind aus der Gruppe, bestehend aus Matratzen und Bettzeug, wobei die Matratzen und das Bettzeug vorzugsweise aus natürlichem oder synthetischem Latex, Kokosfasern, Rosshaar, Stroh, Seegras, Baumwolle, Schur- oder Schafswolle, Seide, Federn, Viskose, Vlies und/oder aus synthetischen Schaumstoffen auf Basis von Polyurethanen bestehen.

12. Verwendung nach einem der Ansprüche 9 bis 11, **dadurch gekennzeichnet, dass** das Lithium-haltige Mittel zum Schutz vor Grabmilben (*Sarcoptes spec*.), Haarbalgmilben (*Demodex spec*.) und Hausstaubmilben (*Dermatophagoides spec*.) dient.

13. Matratzen oder Bettzeug, umfassend eine Lithium-haltige Ausrüstung durch mindestens einen Lithium-haltigen Bestandteil, ausgewählt aus der Gruppe, bestehend aus Lithiumhydroxid; Umsetzungsprodukte, die durch das Verseifen von natürlichen Fetten und Ölen mit Lithiumhydroxid entstehen; Lithiumsalze mineralischer Säuren; Lithiumsalze von Carboxymethylcellulose; Lithium-Salze von Polyethylenglykolcarbonsäuren; Lithiumsalze organischer Säuren der allgemeinen Formel
Li-CO₂-(CH₂)ₙ-H mit n = 0 bis 30,
wobei der Alkylrest der Carbonsäuren geradlinig oder verzweigt, gesättigt oder ungesättigt sowie unsubstituiert oder substituiert sein kann; Lithiumchlorid, Lithiumbromid, Lithiumsulfat, Lithiumnitrat, Lithiumcarbonat und Lithiumphosphat; und Mischungen der vorstehenden Lithium-haltigen Bestandteile.
